# EUROPEAN PATENT APPLICATION

(11) **EP 2 548 553 A1**
(43) Date of publication of application: **23.01.2013**
(21) Application number: 12188621.2
(22) Date of filing: 24.09.2008
(51) Int. Cl.: A61K 31/165, A61K 9/20, A61K 9/28

(54) **GALENICAL FORMULATIONS OF ALISKIREN**

(30) Priority: 28.09.2007 US 975894 P
(62) Divisional of application: 08804674.3
(71) Applicant: Novartis AG, 4056 Basel (CH)
(72) Inventor: Bianchi, Jean-Claude, 68730 Blotzheim (FR); Busies, Heiko, 4123 Allschwil (CH); Meyer, Andreas, 79395 Neuenburg (DE)
(74) Representative: Frigola Deulofeu, Maria Carmen

(57) **Abstract**

The present invention relates to a roller compacted solid oral dosage form comprising a therapeutically effective amount of Aliskiren, or a pharmaceutically acceptable salt thereof, wherein the active ingredient is present in an amount of more than 38% by weight based on the total weight of the oral dosage form, as well as a process of preparing said solid oral dosage form.

## Description

The present invention relates to solid oral dosage forms comprising an orally active renin inhibitor, aliskiren, or a pharmaceutically acceptable salt thereof, as the active ingredient in a suitable carrier medium. In particular, the present invention provides galenic formulations comprising aliskiren, preferably, a hemi-fumarate or nitrate salt thereof, alone or in combination with another active agent. The present invention also relates to the processes for their preparation and to their use as medicaments.

In the following the term "aliskiren", if not defined specifically, is to be understood both as the free base and as a salt thereof, especially a pharmaceutically acceptable salt thereof, most preferably a hemi-fumarate thereof.

Renin released from the kidneys cleaves angiotensinogen in the circulation to form the decapeptide angiotensin I. This is in turn cleaved by angiotensin converting enzyme in the lungs, kidneys and other organs to form the octapeptide angiotensin II. The octapeptide increases blood pressure both directly by arterial vasoconstriction and indirectly by liberating from the adrenal glands the sodium-ion-retaining hormone aldosterone, accompanied by an increase in extracellular fluid volume. Inhibitors of the enzymatic activity of renin bring about a reduction in the formation of angiotensin I. As a result a smaller amount of angiotensin II is produced. The reduced concentration of that active peptide hormone is the direct cause of, e.g., the antihypertensive effect of renin inhibitors. Accordingly, renin inhibitors, or salts thereof, may be employed, e.g., as antihypertensives or for treating congestive heart failure.

The renin inhibitor, Aliskiren, in particular, a hemi-fumarate thereof, is known to be effective in the treatment of reducing blood pressure irrespective of age, sex or race and is also well tolerated. Aliskiren in form of the free base is represented by the following formula 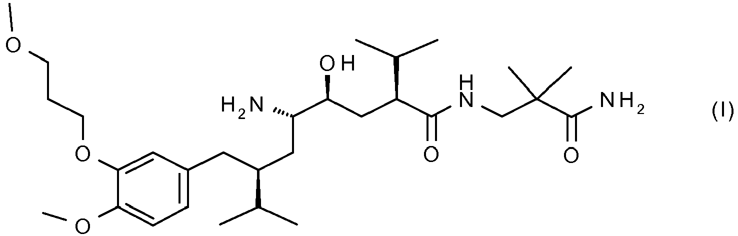 and chemically defined as 2(S),4(S),5(S),7(S)-N-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide. As described above, most preferred is the hemi-fumarate salt thereof which is specifically disclosed in EP 678503 A as Example 83.

Administration of such a pharmaceutical agent via the oral route is preferred to parenteral administration because it allow self-administration by patients whereas parenteral formulations have to be administered in most cases by a physician or paramedical personnel.

However, Aliskiren is a drug substance difficult to formulate due to its physicochemical properties and it is not trivial to make oral formulations in the form of tablets in a reliable and robust way. In addition, in the particular case of high dose of Aliskiren or a pharmaceutically acceptable salt thereof (up to 300 mg of the free base per tablet) makes a high drug loading necessary in order to achieve a reasonable tablet size. So far, only examples of wet granulated Aliskiren formulations have been described and developed which employ a high drug loading of more than 46% by weight based on the total weight of the oral dosage form. Such a solid oral dosage form of Aliskiren is described e.g. in W02005/089729. However, despite their commercial usefulness, wet granulation methods are less preferred since they need the use of granulation liquids and additional drying steps.

Accordingly, a suitable and robust galenical formulation overcoming the above problems related to the properties of Aliskiren needs to be developed.

Surprisingly it has been found that robust galenical formluations comprising aliskiren can be

Thus, present invention relates to a roller compacted solid oral dosage form comprising a therapeutically effective amount of aliskiren, or a pharmaceutically acceptable salt thereof, wherein the active ingredient is present in an amount of more than 38% by weight based on the total weight of the oral dosage form, either dependent on or not dependent on any coating or capsule material used.

Preferably, the amount of the active ingredient in% by weight based on the total weight of the oral dosage form is quoted without taking into account the weight of any coating or capsule used. More preferably, the active ingredient is present in an amount of more than 40% by weight based on the total weight of the oral dosage form.

In a preferred embodiment of the present invention, the active agent, preferably in the form of a salt such as the hemifumarate or the nitrate is present in an amount ranging from 41 to 80%, such as 41 to 60%, by weight based on the total weight of the oral dosage form.

In another preferred embodiment of the present invention, the active agent is present in an amount of more than 42% up to 55% by weight based on the total weight of the oral dosage form. It is particularly preferred that the active agent is present in an amount of more than 48% or even 50% to 80% by weight based on the total weight of the oral dosage form, in order to increase the drug load.

In a solid oral dosage form according to the present invention wherein the active agent consists entirely of aliskiren, or a pharmaceutically acceptable salt thereof, it is preferred if the active agent is present in an amount ranging from about 75 mg to about 600 mg of the free base per unit dosage form.

In a preferred embodiment of the present invention, the active agent consists entirely of aliskiren, or a pharmaceutically acceptable salt thereof, and is present in an amount ranging from about 75 to about 300 mg of the free base per unit dosage form.

In a further preferred embodiment of the present invention, the dosage of aliskiren is in the form of a hemi-fumarate thereof and is present in an amount of about 83, about 166, about 332 or about 663 mg per unit dosage form, i.e. corresponding to 75 mg, 150 mg or 300 mg of the free base per unit dosage form.

Solid oral dosage forms according to the present invention provide for the administration of the active ingredient in a smaller oral form than was heretofore possible for a given unit dose of the active agent. Furthermore, the oral dosage forms obtained are stable both to the production process and during storage, e.g., for about 2 years in conventional packaging, e.g., sealed aluminium blister packs.

The percentages given above and below are based on using a salt such as the hemifumarate or nitrate, and if the free acid or other salts are used, the percentages will be adapted accordingly. For the percentages as used herein, the numbers refer to Aliskiren, thus referring to the free acid or the salt, in particular they refer to the hemifumarate or nitrate.

The terms "effective amount" or "therapeutically effective amount" refers to the amount of the active ingredient or agent which halts or reduces the progress of the condition being treated or which otherwise completely or partly cures or acts palliatively on the condition. The terms "drugs", "active substances", active ingredients", "active agents" etc. as used herein refer to components a) and b) unless specified otherwise. Each of component a) or b) can be referred to as a "drug", "active substance", active ingredient", "active agent" etc..

In the above and in the following the term "Aliskiren", if not defined specifically, is to be understood both as the free base and as a salt thereof, especially a pharmaceutically acceptable salt thereof, such as a hemi-fumarate, hydrogen sulfate, orotate or nitrate, most preferably a hemi-fumarate thereof.

Aliskiren, or a pharmaceutically acceptable salt thereof, can, e.g., be prepared in a manner known *per se,* especially as described in EP 678503 A, e.g., in Example 83.

A solid oral dosage form comprises a capsule or more preferably a tablet or a film-coated tablet.

A solid oral dosage form according to the invention comprises additives or excipients that are suitable for the preparation of the solid oral dosage form according to the present invention. Tabletting aids, commonly used in tablet formulation can be used and reference is made to the extensive literature on the subject, see in particular Fiedler's "Lexicon der Hilfstoffe", 4th Edition, ECV Aulendorf 1996, which is incorporated herein by reference. These pharmaceutically acceptable additives include, but are not limited to, fillers or diluents, binders, disintegrants, lubricants, glidants, stabilising agents, surfactants, film-formers, softeners, pigments and the like. The amount of each additive in a pharmaceutical oral fixed dose combination may vary within ranges conventional in the art.

Suitable fillers include, without limitation, microcrystalline cellulose (e.g., cellulose MK), mannitol, sucrose or other sugars or sugar derivatives, Calcium hydrogen phosphate qualities, starch qualities, preferably corn starch, low-substituted hydroxypropyl cellulose, hydroxyethyl cellulose, hydroxypropyl methyl cellulose, and combinations thereof, preferably, microcrystalline cellulose, e.g., products available under the registered trade marks AVICEL, VIVAPUR, FILTRAK, HEWETEN or PHARMACEL. When present, a filler may be employed in an amount ranging from about 12 % to about 50 %, preferably from about 10% to about 45%, more preferably from about 15% to about 40% by weight of the dosage form (prior to any optional film coating). Preferably, due to the possibility to achieve a high drug load with the roller compaction, a filler may be employed in an amount ranging from about 3 % to about 50 %, preferably from about 5% to about 45%, more preferably from about 7% to about 40% by weight of the dosage form (prior to any optional film coating). The filler may be contained in the internal as well as in the external phase, preferably at least in the internal phase. Preferably, the amount of filler is kept relatively low compared to typical mixtures undergoing roller compaction.

Suitable binders include, without limitation, polyvinylpyrrolidone (PVP), such as e.g., PVP K 30 or PVP90F, polyethylene glycols (PEG), e.g., PEG 4000, hydroxypropylmethyl cellulose, hydroxypropyl cellulose, pregelatinized starch and combinations thereof. Due to its physicochemical properties, microcrystalline cellulose (e.g., cellulose MK), acts as a "dry" binder and could be considered as a binder. However for the purposes of the present invention, microcrystalline cellulose is classed as a filler and not a binder. When present and not considering microcrystalline cellulose also as a binder, a binder may be employed in the internal phase in an amount ranging from about 0.01 % to about 10%, preferably from about 0.1 % to about 5%, by weight of the dosage form (prior to any optional film coating). Preferably, no additional binder to microcrystalline cellulose is being used.

Suitable lubricants include, without limitation, magnesium stearate, aluminum or calcium silicate, stearic acid, CUTINA (Hydrogenated Castor Oil), PEG 4000-8000, talc, glyceryl behenate, sodium stearyl fumarate (PRUV) and combinations thereof, preferably magnesium stearate. When present, a lubricant may be employed in an amount ranging from about 0.1 % to about 5%, preferably from about 0.5% to about 4%, more preferably from about 1.1 % to about 3.3% by weight of the dosage form (prior to any optional film coating) In one embodiment, a lubricant may be employed in an amount ranging from about 0.1 % to about 8%, preferably from about 0.5% to about 6%, more preferably from about 1% to about 6% by weight of the dosage form (prior to any optional film coating). Preferably, the dosage form contains a lubricant both in the external and the internal phase.

Suitable disintegrants include, without limitation, carboxymethylcellulose calcium (CMC-Ca), carboxymethylcellulose sodium (CMC-Na), crosslinked PVP (e.g. CROSPOVIDONE, POLYPLASDONE or KOLLIDON XL), alginic acid, sodium alginate and guar gum, most preferably crosslinked PVP (PVP XL, CROSPOVIDONE), crosslinked CMC (Ac-Di-Sol), carboxymethylstarch-Na (PIRIMOJEL and EXPLOTAB) or combinations thereof. Most preferred disintegrants are crosslinked PVP, preferably PVPP XL and/or crosslinked CMC (Crosscarmellose Sodium, Vivasol, AC-Di-Sol). When present, the disintegrant(s) may be employed in an amount ranging from about 5 % to about 30 %, preferably from about 10 % to about 25 %, by weight of the dosage form (prior to any optional film coating). Depending on the composition and the other additives, disintegrants may be contained in the internal phase only or in the internal as well as in the external phase.

Suitable glidants include, without limitation, colloidal silicon dioxide (e.g., Aerosil 200), magnesium trisilicate, powdered cellulose, talc and combinations thereof. Most preferred is colloidal silicon dioxide. When present and considering starch not as a glidant, a glidant may be employed in an amount ranging from about 0.05% to about 5%, preferably from about 0.1 % to about 3%, such as about 0.1 % to about 1%, by weight of the dosage form (prior to any optional film coating). Depending on the composition and the other additives, glidants may be contained in the internal phase only or in the internal as well as in the external phase. Preferably a glidant is present in order to enhance the flow properties of material undergoing roller compaction.

The solid oral dosage forms of the present invention have a low friability as is not more than 0.8%, preferably not more than 0.6 %. The friability is measured by standard methods known to the person skilled in the art, see the harmonized procedure set forth in the pharmacopeias USP <1216> and EP 2.9.7 and JP.

The solid oral dosage forms of the present invention have also suitable hardness (e.g.an average hardness ranging from about 110 N to about 250 N). Such an average hardness is determined prior to the application of any film coating on the solid dosage form.

In that regard, a preferred embodiment of this invention is directed to the solid oral dosage forms which are film-coated. Suitable film coatings are known and commercially available or can be made according to known methods. Typically film coating materials are hydrophilic polymers such as polyethylene glycol, polyvinylpyrrolidone, polyvinyl alcohol,hydroxypropylcellulose, hydroxymethylcellulose, and hydroxypropylmethylcellulose or the like, of which hydroxypropyl methylcellulose is preferred. The film coating composition ingredients include plasticizers, e.g., polyethylene glycols (e.g. polyethylene glycol 6000), triethylcitrate, diethyl phthalate, propylene glycol, glycerin in conventional amounts, as well as opacifiers such as titanium dioxide, and colorants, e.g. iron oxide, aluminum lakes, etc. Typically, a film coating material is applied in such an amount as to provide a film coating that ranges from about 1 % to about 6% by weight of the solid oral dosage form. Dry mixtures such as Sepifilm or Opadry mixtures prepared by Colorcon Corp. are preferably being used. These products are individually prepared dry premixtures of film forming polymers, opacifiers, colorants and plasticizers which are further processed to aqueous film coating suspensions.

The film coating may be generally applied to achieve a weight increase of the solid oral dosage form of about 1 to 10 wt.%, and preferably about 2 to 6 wt.%.

The film coating can be applied by conventional techniques in a suitable coating pan or fluidized bed apparatus using water and/or conventional organic solvents (e.g., methyl alcohol, ethyl alcohol, isopropyl alcohol), ketones (acetone), etc.

A further embodiment of the present invention is a process for the manufacture of a solid oral dosage form according to the present invention. Such a solid oral dosage form can be prepared by the following method, comprising the steps of roller compacting Aliskiren or a pharmaceutical acceptable salt thereof together with pharmaceutically acceptable additives, optionally mixing with further pharmaceutically acceptable additives, and optionally compressing the final blend into a tablet.

More particularly, the method comprises the steps of
(a) blending Aliskiren or a pharmaceutical acceptable salt thereof and pharmaceutically acceptable additives
(b) sieving the blended Aliskiren or a pharmaceutical acceptable salt thereof and pharmaceutically acceptable additives
(c) blending the sieved material
(d) roller compacting the blended material to form a compacted material;
(e) milling the compacted material to form a milled material referred to as the Aliskiren granulate;
(f) optionally blending the milled material with outer phase, i.e., with pharmaceutically acceptable additives to form a final mixture;
(g) optionally compressing the final blend to form a tablet and
(h) optionally applying a film coat in order to obtain the film coated tablets. Alternatively, the process steps (b) and (f) may be performed in two steps, comprising a first blend with Aliskiren or Aliskiren granulate, resp. and additives without the lubricant and a second (final) blend of the first blend with the lubricant.

Attention is drawn to the numerous known methods of granulating, sieving and mixing employed in the art, e.g., mixing in a free-fall or tumble blender, compressing into tablets on a single-punch or rotary tablet press or compaction on a roller compaction equipment. The sieving step can be accomplished using any suitable means, e.g. using oscillating sieving or hand/vibrating sieves. The blending steps can be accomplished using any suitable means. Typically, Aliskiren or the Aliskiren granulate and pharmaceutically acceptable additives are dispatched to a suitable vessel such as a diffusion blender or diffusion mixer.

The compacting step can be accomplished using any suitable means. Typically, compacting is accomplished using a roller compactor with a compaction force (for development scale machines) ranging from about 1 kN/cm to about 20 kN/cm i.O., preferably about 2 to 10 kN/cm. In one embodiment, a compaction force (for development scale machines) ranging from about 2 kN to about 6 kN/cm i.O., preferably about 3 to 5 kN/cm. is employed. Compaction may also be carried out by slugging the blended powders into large tablets that are then size-reduced. Preferably, the device used is a Freund Corporation; Roller Compactor Type TF Mini. Using this equipment, the screw speed is suitably adjusted to ensure proper quality of the roller compacted material. Preferably, the screw speed is more than 15 rpm, such as 20 to 30 rpm. Moreover, using this equipment, the roll speed is suitably adjusted to ensure proper quality of the roller compacted material. Preferably, the roll speed is 3 to 5 rpm. It is also preferred that no pre-compression force is applied.

The milling/screening step can be accomplished using any suitable means. Typically the compacted material (the "internal phase") is milled through a screening mill or oscillating sieve/mill with a screen of at least 1.0 mesh size, such as 1.0 or 1.2 mm. Preferably the milled material is blended, often with pharmaceutically acceptable additives such as lubricants, fillers, disintegrants and glidants (the "outer phase"), in a diffusion blender.

More preferred for the compaction and the milling/screening is a Gerteis 3-W-Polygran PACTOR e.g. Minipactor 250/25/3 with the following typical characteristics: Gap width 1.0 to 5.0 mm, preferably 2.0 to 4.0 mm, Roll speed 2.5 to 15 rpm, preferably 2.5 to 10 rpm. Mesh size 0.8 to 2.00mm, preferred 1.0 to 1.5mm.

The resulting formulations in accordance with the present invention show the following advantages:
- A relatively high drug loading may easily be achieved;
- The formulation of pharmaceutical oral fixed dose combinations with sufficient hardness, resistance to friability, disintegration time etc. is possible;
- The sticking tendency and poor flow of the drug substance is reduced to a minimum;
- A robust manufacturing process is achieved;
- Scale-up of formulation and process resulting in a reproducible performance is achieved; and
- Sufficient stability to achieve a reasonable shelf life is achieved.
- Shorter processing times are achieved in particular due to the absence of any drying Steps and the process is thus rendered more economic.
- Smaller tablet size while having the same amount of active ingredient
- Lower packaging costs
- Higher number of tablets per batch.

Compared to the prior art wet granuiation, the roller compaction as described herein offers a much more economic process without the use of solvents and additional drying steps and at the same time achieves solid oral dosage forms with a high drug load, preferably even higher than presently prepared with the wet granulation, providing thus overall the possibility to smaller tablet sizes which increases the patient compliance.

The invention likewise relates to a process for the preparation of solid oral dosage forms as described herein above. Such a solid oral dosage form may be produced by working up components as defined herein above in the appropriate amounts, to form unit solid oral dosage forms.

The solid oral dosage forms of the present invention are useful for lowering the blood pressure, either systolic or diastolic or both. The conditions for which the instant invention is useful include, without limitation, hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction (such as Alzheimer's) and stroke, headache and chronic heart failure.

The present invention likewise relates to a method of treating hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure comprising administering to an animal, including human patient, in need of such treatment a therapeutically effective solid oral dosage form according to the present invention.

The present invention likewise relates to the use of a solid oral dosage form according to the present invention for the manufacture of a medicament for the treatment of hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure.

The present invention likewise relates to a pharmaceutical composition for the treatment of hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure, comprising a solid oral dosage form according to the present invention.

Ultimately, the exact dose of the active agent and the particular formulation to be administered depend on a number of factors, e.g., the condition to be treated, the desired duration of the treatment and the rate of release of the active agent. For example, the amount of the active agent required and the release rate thereof may be determined on the basis of known *in vitro* or *in vivo* techniques, determining how long a particular active agent concentration in the blood plasma remains at an acceptable level for a therapeutic effect.

The above description fully discloses the invention including preferred embodiments thereof. Modifications and improvements of the embodiments specifically disclosed herein are within the scope of the following claims. Without further elaboration, it is believed that one skilled in the art can, using the preceding description, utilize the present invention to its fullest extent. Therefore, the Examples herein are to be construed as merely illustrative and not a limitation of the scope of the present invention in any way.

### Example 1: Tablet Formulations & Process Parameters

Compositions of Aliskiren 300 mg (free base) tablets in mg/unit.

| | **Dosage form 1** | **Dosage form 2** | **Dosage form 3** |
|---|---|---|---|
| **Inner Phase** | | | |
| Aliskiren hemi-fumarate | 331.5 (48.8%) | 331.5 (48.8%) | 331.5 (43.4%) |
| Microcrystalline cellulose | 230.1 (33.8%) | 44.1 (6.5%) | 118.05 (15.5%) |
| PVP K 30 | - | - | - |
| Corn starch | - | 180.0 (26.5%) | - |
| Calcium hydrogen phosphate | - | - | 118.05 (15.5%) |
| Crospovidone | 96.4 (14.2%) | 86.4 (12.7%) | 96.4 (12.6%) |
| Aerosil 200 | 2.0 (0.3%) | 2.0 (0.3%) | 2.0 (0.3%) |
| Magnesium stearate | 10.0 (1.5%) | 7.0 (1.03%) | 7.0 (0.9%) |
| **Total Inner Phase** | **670.0** | **651.0** | **673.0** |

| **Outer phase** | | | |
|---|---|---|---|
| Crospovidone | - | 10.0 (1.5%) | - |
| Na Carboxy methylstarch (sodium starch glycollate) | - | - | 76.0 (10.0%) |
| Microcrystalline cellulose | - | 10.0 (1.5%) | - |
| Aerosil 200 | - | 2.0 (0.3%) | - |
| Magnesium stearate | 10.0 (1.5%) | 7.0 (1.03%) | 14.4 (1.9%) |
| **Total weight** | **680.0** | **680.0** | **763.4** |
| Thickness, mean (mm) | 7.2 | 6.7 | 7.0 |
| Hardness, mean (N) | 200 | 200 | 182 |

The components of the Aliskiren tablet were mixed, granulated and compressed as described herein for preparing a roller-compacted Aliskiren tablet using the Freund Corporation; Roller Compactor Type TF Mini with the process parameters described below.

| | **Dosage form 1** | **Dosage Form 2** | **Dosage Form 3** |
|---|---|---|---|
| **Main filler** | **Microcryst. cellulose** | **Corn starch/ Microcryst. cell.** | **Ca- hydrogen phosphate/ Microcryst. cell.** |
| **RC(roller compaction) parameters** | | | |
| Screw speed (rpm) | 22 | 22 | 24 |
| Roll speed | 5 | 5 | 3 |
| Pre compression force (KN) | 0 | 0 | 0 |
| Main compression force (KN) | 3 - 4 | 4.0-4.5 | 3 - 3.2 |

| **Milling/screening parameters** | | | |
|---|---|---|---|
| screen (mm) | 1.0 | 1.0 | 1.2 |

### Example 2: Tablet Formulations & Process Parameters

Compositions of Aliskiren 300 mg (free base) tablets in mg/unit.

| | **Dosage form 4** | | |
|---|---|---|---|
| **Inner Phase** | | | |
| Aliskiren hemi-fumarate | 331.5 (68.4%) | | |
| Microcrystalline cellulose | 35.0 (7.2%) | | |
| Calcium hydrogen phosphate | 35.0 (7.2%) | | |
| Croscarmellose Sodium | 60.0 (12.4%) | | |
| Aerosil 200 | 2.0 (0.4%) | | |
| Magnesium stearate | 9.6 (2.0%) | | |
| **Total Inner Phase** | **473.1** | | |

| **Outer phase** | | | |
|---|---|---|---|
| Aerosil 200 | 2.0 (0.4%) | | |
| Magnesium stearate | 9.6 (2.0%) | | |
| **Total weight** | **484.70** | | |
| Shape | Ovaloid 16x6.3 | | |
| Thickness, mean (mm) | **6** | | |
| Hardness, mean (N) | 175 | | |

The components of the Aliskiren tablet were mixed, granulated and compressed as described herein for preparing a roller-compacted Aliskiren tablet using the Gerteis MINIPACTOR; Roller Compactor with the process parameters described below.

| **Main filler** | **Dosage form 4 Microcryst. cellulose Ca-hydrogen phosphate/** | | |
|---|---|---|---|
| **RC(roller compaction) parameters** | | | |
| Gap (mm) | 3.0 | | |
| Roll speed (rpm) | 2.5 | | |
| Compression force (KN/cm) | 3 | | |
| Automatic Mode | On | | |

| **Milling/screening parameters** | | | |
|---|---|---|---|
| screen (mm) | 1.25 | | |

the sieved material
(d) roller compacting the blended material to form a compacted material;
(e) milling the compacted material to form a milled material referred to as the Aliskiren granulate;
(f) optionally blending the milled material with outer phase, i.e., with pharmaceutically acceptable additives to form a final mixture;
(g) optionally compressing the final blend to form a tablet and
(h) optionally applying a film coat in order to obtain the film coated tablets. Alternatively, the process steps (b) and (f) may be performed in two steps, comprising a first blend with Aliskiren or Aliskiren granulate, resp. and additives without the lubricant and a second (final) blend of the first blend with the lubricant.

## Claims

1. A roller compacted solid oral dosage form comprising a therapeutically effective amount of Aliskiren, or a pharmaceutically acceptable salt thereof, wherein the active ingredient is present in an amount of more than 38% by weight based on the total weight of the oral dosage form.

2. A solid oral dosage form according to claim 1, wherein the active ingredient is present in an amount of more than 40% by weight.

3. A solid oral dosage form according to claim 1 or 2, wherein the active ingredient is present in an amount ranging from 41 to 80%, such as 41 to 60%, by weight.

4. A solid oral dosage form according to any one of the preceding claims, wherein the active ingredient consists entirely of Aliskiren, or a pharmaceutically acceptable salt thereof, and is present in an amount ranging from about 75 to about 300 mg of the free base per unit dosage form.

5. A solid oral dosage form according to any one of the preceding claims, wherein Aliskiren is in the form of a hemi-fumarate thereof, and is present in an amount of about 83, about 166 or about 332 mg per unit dosage form.

6. A solid oral dosage form according to any one of the preceding claims, wherein the dosage form further comprises a filler, preferably in an amount of 3 to 45 %, such as 12 to 45%, by weight of the dosage form.

7. A solid oral dosage form according to claim 6, wherein the filler is microcrystalline cellulose, corn starch and/or calcium hydrogen phosphate

8. A solid oral dosage form according to one any of the preceding claims, wherein the dosage form further comprises a disintegrant, preferably in an amount of 5 to 30% by weight of the dosage form.

9. A solid oral dosage form according to any one of the preceding claims, wherein the dosage form further comprises a lubricant, preferably in an amount of 0.5 to 6%, such as 0.5 to 4%, by weight of the dosage form.

10. A solid oral dosage form according to any one of the preceding claims, wherein the dosage form further comprises a glidant, preferably in an amount of 0.1 to 3.0%, such as 0.1 to 1.0%, by weight of the dosage form.

11. A solid oral dosage form according to Claim 10, wherein the glidant is colloidal silicon dioxide.

12. A solid oral dosage form according to any one of the preceding claims for the treatment of hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, stroke, headache and chronic heart failure.

13. A process for the manufacture of a solid oral dosage form according to any one of claims 1 to 11 comprising the steps of roller compacting Aliskiren, or a pharmaceutical acceptable salt thereof, and pharmaceutically acceptable additives, optionally (after screening) mixing with further pharmaceutically acceptable additives, and optionally compressing the final blend into a tablet.

14. The process according to claim 13 comprising the steps of
(a) blending Aliskiren, or a pharmaceutical acceptable salt thereof, and pharmaceutically acceptable additives;
(b) sieving the blended Aliskiren, or a pharmaceutical acceptable salt thereof, and pharmaceutically acceptable additives;
(c) blending the sieved material;
(d) roller compacting the blended material to form a compacted material;
(e) milling the compacted material to form a milled material referred to as the Aliskiren granulate;
(f) optionally blending the milled material with outer phase, i.e., with pharmaceutically acceptable additives to form a final mixture;
(g) optionally compressing the final blend to form a tablet; and
(h) optionally applying a film coat in order to obtain the film coated tablets,
